Europäisches Patentamt

European Patent Office

Office européen des brevets

(11) Publication number: **0 242 082**
**A2**

# (12) EUROPEAN PATENT APPLICATION

(21) Application number: 87302800.5

(22) Date of filing: 31.03.87

(51) Int. Cl.⁴: **G01N 33/569** , G01N 33/543

(30) Priority: 16.04.86 GB 8609264

(43) Date of publication of application:
21.10.87 Bulletin 87/43

(84) Designated Contracting States:
AT BE CH DE ES FR GB GR IT LI LU NL SE

(71) Applicant: CITY OF LONDON POLYTECHNIC
117-119 Houndsditch
London EC3A 7BH(GB)

(72) Inventor: Gaylarde, Christine Claire
52 Crediton Hill
London NW6(GB)
Inventor: Cook, Paul Edward
13 Cherry Garden Avenue
Folkestone Kent(GB)

(74) Representative: Grundy, Derek George Ritchie
et al
CARPMAELS & RANSFORD 43, Bloomsbury
Square
London WC1A 2RA(GB)

(54) Method of detecting and enumerating sulphate-reducing bacteria.

(57) The invention relates to an ELISA method for detecting and enumerating sulphate-reducing bacteria in a given sample. The method includes either or both of the steps of immobilizing bacterial cells on a support by the application of an immobilizing agent selected from titanous hydroxide suspension, titanic hydroxide suspension and zirconium hydroxide suspension and blocking non-specific binding sites with a blocking agent comprising a casein buffer.

EP 0 242 082 A2

# METHOD OF DETECTING AND ENUMERATING SULPHATE-REDUCING BACTERIA

This invention relates to a method of detecting and enumerating sulphate-reducing bacteria and, in particular, to an enzyme-linked immunosorbent assay (ELISA) method.

Sulphate-reducing bacteria are so called because they obtain their energy by reducing sulphate ions in their environment to sulphide ions. Indeed, it is this capability which makes the presence of sulphate-reducing bacteria in certain environments a problem.

One industry where the presence of sulphate-reducing bacteria in certain areas can be particularly problematical is the oil industry. Sulphate-reducing bacteria inhabit the water base of oil-bearing strata and shales, the drilling muds used in oil exploration and sea water, which is used for filling the legs of oil rigs to increase their stability and for filling ballast tanks. Moreover, the bacteria proliferate in these conditions. However, the sulphide ions produced as a result of sulphate-reducing bacteria reducing the sulphate ions which are commonly present in sea water can cause corrosion to the pipework and platform itself and can "sour" the oil produced and stored on a rig. It is therefore of considerable importance that sulphate-reducing bacteria in the vicinity of an oil rig be eliminated as far as possible and, in order to achieve this, biocides are widely used. However, to check that a biocide has been effective, it is necessary to take samples from various parts of a rig and then determine the number of sulphate-reducing bacteria, if any, in each sample.

Until recently, the most commonly used method for detecting and enumerating sulphate-reducing bacteria was a most probable number test which involved taking a number of serum bottles containing 9ml Postgate's B medium or another suitable medium which had been capped under an inert gas atmosphere and sterilized, inoculating a first bottle with 1ml of sample using a sterile disposable syringe, agitating the first bottle vigorously and then removing 1ml from this bottle for inoculation into a second bottle. This dilution procedure was repeated until a dilution of $10^6$ was reached and three to five replicates of each dilution were prepared. The bottles were then incubated at a temperature within 5°C of the environmental temperature for 14 days. Any bottles which showed blackening of the contents within this time were considered positive, i.e. bacteria present. The numbers of sulphate-reducing bacteria present were then estimated by referring to most probable number tables.

The above method is recommended by the National Association of Corrosion Engineers task group. However, it is also possible to use similar most probable number tests using different liquid media. All such most probable number tests using liquid media work in the same way, that is, the liquid growth medium contains an appropriate component which changes colour in response to by-products produced by sulphate-reducing bacteria, thereby enabling positive samples to be identified. However, such methods have a number of disadvantages.

One of the main problems of such methods is that sulphate-reducing bacteria are notoriously difficult to grow thus not all viable bacteria in the sample may survive to produce sufficient by-products to allow detection and non-viable bacteria present will not be detected at all. Also, because of the difficulties involved in growing sulphate-reducing bacteria, it may take several days for the bacteria to produce sufficient by-products to cause a colour change and, indeed, it is recommended that the containers be kept for a month before a negative result, i.e. total absence of sulphate-reducing bacteria, can be declared. This makes the method very time-consuming and of little practical use where day-to-day monitoring is required. Moreover, not all varieties of sulphate-reducing bacteria will grow in one particular growth medium which means that the number of bottles showing a positive colour change will almost certainly be lower than it should be. This method also suffers from a problem associated with all most probable number tests of this type in that the number of bacteria present is ultimately determined from standard probability tables on the basis of the number of positive results obtained at all dilutions and therefore, if an abnormally low number of positive results is obtained at one dilution, the final number will be reduced even though the low number of positive results may have been a complete aberration. Indeed, the fact that sulphate-reducing bacteria are notoriously difficult to grow makes this test even more susceptible to error on this basis.

Clearly, it would be desirable to provide a method of detecting and enumerating sulphate-reducing bacteria that does not suffer from the above disadvantages, that is, a method which is relatively quick and allows direct determination of the number of sulphate-reducing bacteria present, regardless of variety or viability.

Over the last ten years, ELISA techniques have been developed which have enabled various bacteria to be detected and studied much more easily than by conventional methods and quantitative ELISA techniques have been devised for the enumeration of such bacteria. A typical quantitative ELISA method involves applying various dilutions of a sample and standards containing a known number of bacterial cells to a suitable support, e.g. a microtitre plate, immobilizing the cells on the support, applying an antiserum to the bacteria in question, applying an antibody conjugated to an enzyme, applying an enzyme substrate, applying a suitable reaction terminator and then determining the absorbance of the sample and standards at a selected wavelength in e.g. a microtitre plate reader.

Such quantitative ELISA methods have been shown to have sensitivities and specificities comparable to radioimmunometric assays and immunofluorescence assays whilst offering several advantages over such methods. However, because of the time required to perform the necessary incubation and washing steps between the steps outlined above, such methods still typically take about two days to perform. Moreover, the accuracy of the results obtained is greatly affected by the efficiency of the immobilization of cells to the support, since any cells which are not immobilized will simply be washed away in the first washing stage thereby reducing the number of cells which are detected in the assay and producing an artificially low result. The accuracy of the results is also affected by the occurrence of non-specific binding of the antiserum and second antibody (conjugated to an enzyme) to the immunogen and directly to the support. Blocking agents, such as Tween 20, have been used in the diluent buffers and washing agents in an attempt to block these non-specific binding sites. However, such conventional blocking agents have proved far from completely effective.

According to the present invention there is provided an ELISA method of detecting and enumerating sulphate-reducing bacteria in a given sample which includes

(a) immobilizing bacterial cells on a support by the application of an immobilizing agent selected from titanous hydroxide suspension, titanic hydroxide suspension and zirconium hydroxide suspension; and/or

(b) blocking non-specific binding sites by treating immobilized cells with a blocking agent comprising a casein buffer.

It is particularly preferred that titanous hydroxide suspension be used as the immobilizing agent and it is also preferred that the volume of immobilizing agent used in respect of each sample be one fifth the volume of the sample.

Preferably, the casein buffer consists of 10mM/l Tris (hydroxymethyl) aminomethane in hydrochloride of pH 7.6 containing 154 mM/l sodium chloride and 0.5% (w/v) casein. The casein buffer may also contain the antimicrobical agent Thimerosal (Sigma Chemical Company Ltd., Poole, Dorset), preferably in a concentration of 0.02% (w/v).

It is also preferred that the support is a microtitre plate.

The use of a titanous hydroxide suspension, a titanic hydroxide suspension or a zirconium hydroxide suspension as the immobilizing agent for sulphate-reducing bacteria cells has been found to be far more efficient than conventional immobilizing agents. In this respect, titanous hydroxide has proved to be particularly advantageous in that it has been shown to immobilize approximately 95% of the bacterial cells present in samples. Moreover, because of the greater efficiency of these immobilizing agents, the periods of incubation required at various stages of the procedure may be considerably reduced in length as compared to conventional ELISA techniques. For instance, when titanous hydroxide is used, the initial incubation period immediately following application of the immobilizing agent is only 30 minutes whilst overnight incubation is normally required in conventional ELISA methods.

Initial experiments, in which conventional ELISA techniques using conventional reagents were applied to sulphate-reducing bacteria, suffered from very high non-specific binding, which produced high background absorbance readings. However, the use of a casein buffer, which although known per se has never been used previously for ELISA techniques involving bacteria, has been found largely to eliminate this problem. It is thought that a casein buffer is a particularly effective blocking agent because it is heterogeneous and it is therefore capable of blocking more non-specific binding sites than conventional blocking agents.

A significant improvement in the accuracy of the results as compared to conventional ELISA techniques may be achieved either solely by the use of a titanous, titanic or zirconium hydroxide suspension as immobilizing agent or solely by the use of a casein buffer as a blocking agent. However, the use of both these reagents together has been found to be particularly advantageous.

An embodiment of the invention is illustrated in the following Example.

## EXAMPLE

### (a) Preparation of materials

Fifteen strains of sulphate-reducing bacteria were obtained from the National Collection of Industrial Bacteria (NCIB), Torry Research Station, PO Box 31, 135 Abbey Road, Aberdeen, Scotland, AB9 8DG. These are listed in the following table:-

| No. | Strain | Accession No. |
|-----|--------|---------------|
| 1 | Desulfovibrio vulgaris subspecies vulgaris (Woolwich) | NCIB No.8457 |
| 2 | Desulfovibrio vulgaris subspecies vulgaris (Hildenborough) | NCIB No.8303 |
| 3 | Desulfovibrio vulgaris subspecies vulgaris (Holland D6) | NCIB No.8311 |
| 4 | Desulfovibrio vulgaris subspecies vulgaris (Marburg) | NCIB No.11727 |
| 5 | Desulfovibrio vulgaris subspecies vulgaris (F2) | NCIB No.11564 |
| 6 | Desulfovibrio vulgaris subspecies vulgaris (S6) | NCIB No.11565 |
| 7 | Desulfovibrio gigas | NCIB No.9332 |
| 8 | Desulfovibrio salexigens (New Jersey SW8) | NCIB No.8315 |

(List of Sulphate-reducing bacteria strains used - cont.)

| No. | Strain | Accession No. |
|-----|--------|---------------|
| 9 | Desulfovibrio salexigens (California 43:63) | NCIB No.8364 |
| 10 | Desulfovibrio desulfuricans subspecies desulfuricans (Essex 6) | NCIB No.8307 |
| 11 | Desulfovibrio desulfuricans subspecies desulfuricans (New Jersey Sol C-1) | NCIB No.8313 |
| 12 | Desulfovibrio desulfuricans subspecies desulfuricans (California HC 29.137.11) | NCIB No.8339 |
| 13 | Desulfovibrio desulfuricans subspecies aestuarii (Sylt 3) | NCIB No.9335 |
| 14 | Desulfovibrio vulgaris subspecies oxamicus (Monticello 2) | NCIB No.9442 |
| 15 | Desulfotomaculum nigrificans (Teddington garden) | NCIB No.8351 |

Each strain was then used to prepare a homologous sulphate-reducing bacteria antigen solution as follows. First the strains were grown in Postgate's medium C (Postgate, 1984) in batch culture. After five days, the cells were harvested by centrifugation at 2600xg for 30 minutes at 4°C and twice resuspended in phosphate buffered saline (PBS), containing 0.14 M/l sodium chloride and 25 mM/l phosphate buffer of pH 7.4, and centrifuged. The pellet of cells obtained was then resuspended in PBS and a small subsample was taken from which the concentration of cells was estimated using a haemocytometer. The concentration of cells was then adjusted to approximately $10^9$ cells per ml with PBS.

The fifteen strains of sulphate-reducing bacteria listed above were also used to raise antisera in New Zealand white rabbits as follows. First, the rabbits were bled for pre-immune serum and then they were injected with 1ml of a suspension of a single strain of sulphate-reducing bacteria cells, having a concentration of $10^9$ cells per ml in PBS, mixed with an equal volume of Freund's Complete Adjuvant. The rabbits were reinoculated 10 days and 20 days after the initial injection, but with the cell suspension mixed with Freund's Incomplete Adjuvant. The rabbits were bled after 27 days and thereafter at weekly intervals. If the level of antibody obtained was declining, the rabbit was given a further injection of cells in Freund's incomplete adjuvant and then bled at weekly intervals. All bleeds were obtained from the marginal ear vein. The blood samples were left to clot overnight at 4°C and the serum then collected and stored at -20°C.

The antibody titre of each antiserum was determined by whole cell agglutinations in microtitre plates as follows. First, 50µl of PBS were added to each well. Then each antiserum was serially diluted two-fold twenty-one times and the undiluted antiserum and each dilution were individually and separately added to twenty-two successive wells thereby producing a twenty-two well series of increasing dilution of antiserum. 50µl of the homologous sulphate-reducing bacteria antigen suspension, prepared as described above, having a concentration of $10^9$ cells per ml in PBS were then added to each well and the plates covered and incubated at 4°C overnight. The plates were then inspected and the antibody titre calculated as being the reciprocal of the last two-fold dilution showing agglutination.

Polyvalent cocktail antisera were then produced by adjusting the agglutination titre of the antiserum of each strain to 4096 and mixing the antisera of selected strains in equal quantity.

A suspension of titanous hydroxide was prepared by adding 40ml distilled water to 2g titanous chloride and then adding 5M ammonia solution dropwise with continuous agitation until a pH value of 7.0-7.2 was reached. The titanous hydroxide suspension produced was then washed three times with 1000ml of 0.85% (w/v) saline each time by mixing and aspiration and the resultant suspension stored in a screw cap bottle in the dark until required.

## (b) ELISA method

The samples to be tested were extracted by centrifugation and re-suspended in 0.85% (w/v) sodium chloride solution. The samples and standards were then diluted to various levels in 0.85% (w/v) sodium chloride solution and applied to the wells of microtitre plates. In order to immobilize the cells, 50µl of the titanous hydroxide suspension were added for each 100µl of sample or standard applied to the wells and the plates were then shaken for 30 minutes at room temperature (20-24°C). The plates were shaken in order to keep the titanous hydroxide in suspension thereby increasing the efficiency of the reaction. After this incubation period, the plates were washed three times, each for a period of three minutes, in casein buffer which consists of 10mM/l Tris (hydroxymethyl) aminomethane hydrochloride (Tris-HCl) of pH 7.6 containing 154mM/l sodium chloride and 0.5% (w/v) casein (Hammarsten grade).

A polyvalent antiserum to strains 1, 2, 9, 10, 11, 13 and 14 of the sulphate-reducing bacteria listed above was taken and diluted 1:250 in casein buffer. 150µl of the diluted antiserum was then applied to wells of each plate and the plates were incubated at 55°C for 90 minutes. After incubation, the plates were again washed in casein buffer for three periods of three minutes.

Goat anti-rabbit IgG conjugated to horse radish peroxide (Sigma Chemical Company Ltd., Poole, Dorset) was diluted 1:500 in casein buffer and 150µl of this solution were added to wells of the plates. The plates were then incubated at 37°C for 2 hours. After incubation, the plates were washed four times, each for a period of three minutes, in PBS containing 0.14M/l sodium chloride and 25mM/l phosphate buffer of pH 7.4. The casein buffer could not be used for this final washing stage because the casein buffer is colloidal and this would therefore interfere with the absorbance reading in the final stage of the method (see below).

A suitable enzyme substrate was then prepared by dissolving 1.009g of 3, 3', 5, 5' - tetramethylbenzidene in 100ml of dimethylsulphoxide, adding 10ml of this solution to 1 litre of 0.1M/l sodium acetate-citric acid buffer of pH 5.5 and adding sufficient hydrogen peroxide to produce a concentration of 1.3mM/l of hydrogen peroxide in the final solution. 200µl of this was then added to the plates and the plates incubated for one hour at room temperature to allow a colour to develop.

50µl of 12.5% (v/v) sulphuric acid was then added to the plates to terminate the reaction. The solutions in each plate were then transferred to a clean, unused plate and the absorbance of the samples and standard solutions read at a wavelength of 450nm using a microtitre plate reader. Transfer of the test solutions to a clean plate was necessary because the titanous hydroxide suspension used for immobilization of the cells tends to form a film on the wells which can interfere with the absorbance reading.

The number of sulphate-reducing bacteria in the sample solutions was determined by comparing the absorbance of the sample solutions with that of the standard solutions.

The ELISA method of the invention illustrated in the above Example enables as few as 50 organisms per ml of sample to be detected and enumerated and can be performed in six hours thus enabling the test to be carried out within a single working day even with initial extraction of the samples.

Thus, the method of the invention is quicker and more accurate than conventional ELISA techniques and far quicker and much more sensitive than the conventional most probable number test.

It will of course be understood that the present invention has been described above purely by way of example and modifications of detail can be made within the scope of the invention. For instance, although the method described in the Example utilizes whole cells of sulphate-reducing bacteria, the method could be performed equally well using sonicated cells.

**Claims**

1. An ELISA method of detecting and enumerating sulphate-reducing bacteria in a given sample which includes

(a) immobilizing bacterial cells on a support by the application of an immobilizing agent selected from titanous hydroxide suspension, titanic hydroxide suspension and zirconium hydroxide suspension; and/or

(b) blocking non-specific binding sites by treating the support with a blocking agent comprising a casein buffer.

2. An ELISA method according to claim 1 in which the immobilizing agent is titanous hydroxide suspension.

3. An ELISA method according to claim 1 or claim 2 in which the volume of immobilizing agent used in respect of each sample is half the volume of the sample.

4. An ELISA method according to any one of claims 1, 2 and 3 in which the casein buffer consists of 10mM/l Tris (hydroxymethyl) aminomethane hydrochloride of pH 7.6 containing 154mM/l sodium chloride and 0.5% (w/v) casein.

5. An ELISA method according to any one of claims 1, 2 and 3 in which the casein buffer contains Thimerosal.

6. An ELISA method according to claim 5 in which the concentration of Thimerosal in the casein buffer is 0.02% (w/v).

7. An ELISA method according to any preceding claim in which the support is a microtitre plate, tube, bead mesh, stick or pad.

8. An ELISA method substantially as hereinbefore described in part (b) of the Example.